# EUROPÄISCHE PATENTANMELDUNG

(11) **EP 4 129 966 A1**
(43) Veröffentlichungstag der Anmeldung: **08.02.2023**
(21) Anmeldenummer: 21020396.4
(22) Anmeldetag: 04.08.2021
(51) Int. Cl.: C07C 41/01, C07C 41/09, C07C 43/04, C07C 29/151, C07C 31/04

(54) **VERFAHREN UND ANLAGE ZUR HERSTELLUNG VON DIMETHYLETHER**

(71) Anmelder: Linde GmbH, 82049 Pullach (DE)
(72) Erfinder: Wawrzinek, Klemens, 82049 Pullach (DE); Peschel, Andreas, 82049 Pullach (DE); Beneke, Enette, 82049 Pullach (DE)
(74) Vertreter: Fischer, Werner

(57) **Zusammenfassung**

Die Erfindung betrifft ein Verfahren zur Herstellung eines Dimethylether enthaltenden Produktstroms (4), wobei ein zumindest Wasserstoff und ein oder mehrere Kohlenstoffoxide aus der Gruppe von Kohlenstoffmonoxid und Kohlenstoffdioxid enthaltender Eduktstrom (2) zumindest teilweise unter Erhalt eines Dimethylether, Kohlenstoffdioxid, Methanol und Wasser enthaltenden Rohproduktstroms (3) einer direkten katalytischen Umsetzung (R, R01, R02) zu Dimethylether unterworfen wird, und wobei der Produktstrom (4) unter Verwendung einer Produktaufbereitung (T, T10, T01, T02, T03) gebildet wird, der zumindest ein Teil des Rohproduktstroms (3) zugeführt wird, dadurch gekennzeichnet, dass der Produktstrom (4) als zumindest Dimethylether und Kohlenstoffdioxid enthaltender Flüssigstrom gebildet wird, und dass der Produktstrom (4) unter Verwendung zumindest eines Teils einer Sumpfflüssigkeit einer in der Produktaufbereitung (T, T10, T01, T02, T03) verwendeten Trennkolonne (T02) bereitgestellt wird. Ferner wird eine Anlage zur Durchführung eines solchen Verfahrens vorgeschlagen.

## Beschreibung

Die Erfindung betrifft einen Reaktor und ein Verfahren zur Herstellung eines Dimethylether enthaltenden Produktgemischs aus Synthesegas.

### Hintergrund der Erfindung

Bei Dimethylether (DME) handelt es sich um den strukturell einfachsten Ether. Dimethylether enthält zwei Methylgruppen als organische Reste. Dimethylether ist polar und findet herkömmlicherweise in flüssiger Form als Lösungsmittel Verwendung. Dimethylether kann ferner als Kühlmittel eingesetzt werden und herkömmliche Fluorchlorkohlenwasserstoffe ersetzen.

Neuerdings wird Dimethylether zunehmend als Ersatz für Brenngas (Flüssiggas) und herkömmliche Kraftstoffe wie Diesel eingesetzt. Aufgrund seiner vergleichsweise hohen Cetanzahl von 55 bis 60 müssen beispielsweise herkömmliche Dieselmotoren für den Betrieb mit Dimethylether nur geringfügig modifiziert werden. Dimethylether verbrennt vergleichsweise sauber und ohne Rußbildung. Wird Dimethylether aus Biomasse hergestellt, gilt er als sogenannter Biokraftstoff und kann daher steuerbegünstigt vermarktet werden. Allerdings kann Dimethylether auch für andere Zwecke verwendet werden, beispielsweise als Ausgangsstoff für chemische Synthesen. Somit kann er zur Decarbonisierung der chemischen Industrie beitragen, wenn DME aus nichtfossilen Quellen gewonnen wird.

Dimethylether kann entweder direkt aus Methanol oder indirekt aus Synthesegas, z.B. erzeugt aus Erd- oder Biogas, produziert werden. Synthesegas ist ein Gasgemisch, das zumindest Kohlenstoffmonoxid und Wasserstoff in wechselnden Anteilen, die zusammengenommen jedoch den überwiegenden Teil des Gasgemischs ausmachen, enthält. Synthesegas kann auch mittels anderer Verfahren, beispielsweise durch Pyrolyse von Kohle, Öl, kohlenstoffhaltigen Abfällen, Biomasse oder anderen kohlenstoffhaltigen Ausgangsstoffen, durch Trockenreformierung (DryReforming) von Erdgas mit Kohlenstoffdioxid, durch Dampfreformierung (Steam Reforming) von Erdgas, durch autotherme Reformierung (ATR) von Erdgas, durch partielle Oxidation (POX) von Kohlenwasserstoffen, insbesondere Erdgas bzw. Methan, oder Kombinationen aus den genannten Verfahren, gewonnen werden. Das Synthesegas wird anschließend entweder in einer zweistufigen Reaktion zu Methanol und anschließend zu Dimethylether oder in einer einstufigen Reaktion direkt zu Dimethylether konvertiert.

Die Synthese von Dimethylether aus Synthesegas ist thermodynamisch und wirtschaftlich vorteilhaft gegenüber einer Synthese aus Methanol.

Unter Synthesegas (kurz Syngas) wird im Rahmen dieser Erfindung jedes Fluid verstanden, das zu einem überwiegenden Anteil aus Wasserstoff und Kohlenstoffmonoxid gebildet ist, unabhängig von der Herkunft eines solchen Fluides. Synthesegas im Sinne dieser Erfindung kann auch nicht unerhebliche Anteile an Kohlenstoffdioxid aufweisen.

Die vorliegende Erfindung betrifft insbesondere die einstufige Synthese von Dimethylether, wobei unter einer "einstufigen" Synthese eine Synthese verstanden wird, bei der sämtliche Reaktionen in einunddemselben Reaktor und in einunddemselben Katalysatorbett (aber nicht notwendigerweise an einunddemselben Katalysatorpartikel, also z.B. auch einem Katalysatorbett, das sowohl einen Katalysator für die Methanolsynthese als auch einen Katalysator für die Dehydratisierung von Methanol zu Dimethylether enthält) ablaufen. Nachfolgend wird für eine einstufige Synthese von Dimethylether auch der Begriff "Direktsynthese" verwendet. Bei den ablaufenden Reaktionen handelt es sich um eine Reaktion von Wasserstoff mit Kohlenstoffmonoxid und/oder Kohlenstoffdioxid zu Methanol und eine (Weiter-) Reaktion von Methanol zu Dimethylether und Wasser. Die einstufige Synthese von Dimethylether ist beispielsweise aus der US 4,536,485 A und der US 5,189,203 A bekannt. Herkömmlicherweise werden hierbei Hybridkatalysatoren verwendet. Die Reaktion ist exotherm und erfolgt typischerweise bei einer Temperatur von 200 bis 300 °C und bei einem Druck von 20 bis 100 bar.

Zur einstufigen Synthese von Dimethylether werden normalerweise aufrechtstehende Rohrreaktoren eingesetzt, die von unten mit druckbeaufschlagtem und erhitztem Synthesegas beschickt werden. Ein in dem Rohrreaktor erhaltener Produktstrom wird kopfseitig entnommen, gekühlt und einer Trennung zugeführt.

Der Produktstrom enthält typischerweise neben Dimethylether nicht umgesetzte Komponenten des Synthesegases sowie weitere Reaktionsprodukte. Typischerweise weist der Produktstrom neben Dimethylether zumindest Methanol, Wasser, Kohlenstoffdioxid, Kohlenstoffmonoxid und Wasserstoff sowie in geringerer Menge Methan, Ethan, organische Säuren und höhere Alkohole auf.

In einem Gasgemisch, das aus dem Produktstrom gebildet wird, sind damit typischerweise neben Dimethylether noch Kohlenstoffdioxid und leichter als Kohlenstoffdioxid siedende Komponenten wie Wasserstoff und Kohlenstoffmonoxid enthalten. Je nach Produktspezifikation müssen diese zumindest teilweise abgetrennt werden.

Die Zusammensetzung des Produktstroms (dieser oder ein hieraus gebildetes Gasgemisch wird nachfolgend auch als "Dimethylether enthaltendes Produktgemisch" bezeichnet) wird dabei insbesondere durch die gewählten Reaktionsbedingungen während der Synthese bestimmt. Je näher diese Zusammensetzung stromab des Reaktors an der geforderten Spezifikation liegt, desto geringer ist der Aufwand, der zur Aufreinigung des Produktstroms betrieben werden muss.

Insbesondere in Bezug auf die hier verwendete Terminologie, aber auch auf grundsätzliche Überlegungen die Synthese von Dimethylether aus Synthesegas betreffend, sei auf den Artikel von I. Kiendl et al., Chem. Ing. Tech. 2020, 92, No. 6, 736-745, verwiesen.

In einigen Anwendungsfällen darf bzw. soll ein entsprechendes Produktgemisch auch nennenswerte Mengen an Kohlenstoffdioxid enthalten. Dafür soll daher ein verbessertes Reaktorkonzept für die einstufige Synthese von Dimethylether aus Synthesegas angegeben werden.

### Offenbarung der Erfindung

Diese Aufgabe wird durch Verfahren und Anlagen gemäß den jeweiligen unabhängigen Patentansprüchen gelöst. Vorteilhafte Ausgestaltungen und Weiterbildungen sind Gegenstand der Unteransprüche sowie der nachfolgenden Beschreibung.

Vor der Erläuterung der Merkmale und Vorteile der vorliegenden Erfindung werden deren Grundlagen und die verwendeten Begriffe erläutert.

Ein Fluid (die Bezeichnung "Fluid" wird nachfolgend auch kurz für entsprechende Ströme, Fraktionen usw. verwendet) ist von einem anderen Fluid (das auch als Ausgangsfluid bezeichnet wird) "abgeleitet" oder aus einem solchen Fluid "gebildet", wenn es zumindest einige in dem Ausgangsfluid enthaltene oder aus diesem erhaltene Komponenten aufweist. Ein in diesem Sinne abgeleitetes bzw. gebildetes Fluid kann aus dem Ausgangsfluid durch Abtrennen oder Abzweigen eines Anteils oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten bzw. gebildet werden. Ein Strom kann auch beispielsweise einfach dadurch "gebildet" werden, dass er aus einem Speicherbehälter abgezogen wird.

Fluide können im hier verwendeten Sprachgebrauch reich oder arm an einer oder mehreren enthaltenen Komponenten sein, wobei "reich" für einen Gehalt von wenigstens >50%, 75%, 90%, 95%, 99%, 99,5%, 99,9%, 99,99% oder 99,999% und "arm" für einen Gehalt von höchstens 10%, 5%, 1%, 0,1%, 0,01% oder 0,001% auf molarer, Gewichts- oder Volumenbasis stehen kann. Auch Fluide, in denen kein Einzelbestandteil einen Anteil von mehr als 50% erreicht, kann "reich" an einer Komponente sein. Dies bezieht sich in einem solchen Fall auf die Komponente mit dem größten Anteil an dem Fluid. Fluide können im hier verwendeten Sprachgebrauch angereichert oder abgereichert an einer oder mehreren Komponenten sein, wobei sich diese Begriffe auf einen entsprechenden Gehalt in einem Ausgangsfluid beziehen, aus dem das Fluid gebildet wurde. Das Fluid ist "angereichert", wenn es zumindest den 1,1-fachen, 1,5-fachen, 2-fachen, 5-fachen, 10-fachen 100-fachen oder 1.000-fachen Gehalt, "abgereichert", wenn es höchstens den 0,9-fachen, 0,5-fachen, 0,1-fachen, 0,01-fachen oder 0,001-fachen Gehalt einer entsprechenden Komponente, bezogen auf das Ausgangsfluid, enthält. Ein "überwiegend" eine oder mehrere Komponenten enthaltendes Fluid enthält diese eine oder mehreren Komponenten zu wenigstens >50%, 75%, 90%, 95%, 98% oder 99% bzw. ist reich an diesen.

Ist nachfolgend kurz von einer "Synthese" von Dimethylether die Rede, wird hierunter ein Verfahren verstanden, bei dem ein Synthesegas bzw. Syngas enthaltender Einsatz, also ein Gasgemisch, das in geeigneten Anteilen zumindest Kohlenstoffmonoxid und Wasserstoff sowie ggf. auch Kohlenstoffdioxid enthält, zu einem entsprechenden Dimethylether enthaltenden Produktstrom umgesetzt wird. Der Anteil an Wasserstoff in dem Synthesegas kann dabei beispielsweise unter Verwendung einer WassergasShift-Reaktion erhöht werden. Dabei reagiert in dem Synthesegas vorhandenes Kohlenstoffmonoxid mit zu diesem Zweck zugegebenem Wasser(dampf) zu Kohlenstoffdioxid, das gegebenenfalls abgetrennt werden kann. Ein derart nachbehandeltes Synthesegas wird auch als geshiftetes Synthesegas bezeichnet.

Generell sollen hier unter einer "Wassergasshift" immer beide Richtungen der mit diesem Begriff bezeichneten Gleichgewichtsreaktion verstanden werden, also die Reaktion von Kohlenstoffmonoxid mit Wasser zu Kohlenstoffdioxid und Wasserstoff und umgekehrt (letztere auch als "reverse" Wassergasshift, RWGS, bezeichnet). Die Richtung der Wassergasshift wird dem Bedarf entsprechend eingesetzt.

Ein der Synthese unmittelbar unterworfener Einsatzstrom heißt im Rahmen dieser Erfindung "Eduktstrom", ohne Präjudiz auf dessen konkrete Herkunft. Insbesondere kann ein solcher Eduktstrom neben Frischeinsatzkomponenten auch, beispielsweise von stromab der Synthese, rezyklierte Komponenten enthalten.

Ein entsprechender Produktstrom der Synthese von Dimethylether enthält aufgrund der nicht vollständigen Reaktion und aufgrund des Auftretens von Nebenreaktionen bei der Synthese, insbesondere in Abhängigkeit von den Charakteristika der verwendeten Katalysatoren und den jeweiligen Gehalten der Komponenten des Synthesegases, nicht ausschließlich Dimethylether, sondern weitere Verbindungen. Diese sind zumindest Methanol, Wasser, Kohlenstoffdioxid, Kohlenstoffmonoxid und Wasserstoff, jedoch typischerweise auch geringere Mengen Methan, Ethan, organische Säuren und höhere Alkohole. Diese genannten weiteren Verbindungen müssen, wie erwähnt, abgetrennt oder zumindest abgereichert werden. Die Abtrennung erfolgt einerseits, um nachfolgende Trennschritte zu ermöglichen, und andererseits, um Dimethylether in der geforderten Reinheit, also "spezifikationsgerecht" zu gewinnen. Wie ebenfalls bereits erwähnt, ist diese Abtrennung umso unproblematischer, je reiner der Produktstrom die Reaktion verlässt.

Charakteristische Kenngröße für die Zusammensetzung des Synthesegases ist die Stöchiometriezahl (engl. Stoichiometric Number oder Stoichiometric Module) SN, die gemäß folgender Gleichung definiert ist. Die SN setzt die Konzentrationen von Wasserstoff, Kohlenstoffdioxid und Kohlenstoffmonoxid ins Verhältnis und sollte für die Methanol-Synthese typischerweise bei mindestens 2,0 liegen, wie auch in der erwähnten Publikation von Kiendl et al. erläutert.

SN = ([H₂]-[CO₂]) / ([CO]+[CO₂])

### Merkmale und Vorteile der Erfindung

Bei einem erfindungsgemäßen Verfahren zur Herstellung eines Dimethylether enthaltenden Produktstroms wird ein zumindest Wasserstoff und ein oder mehrere Kohlenstoffoxide aus der Gruppe von Kohlenstoffmonoxid und Kohlenstoffdioxid enthaltender Eduktstrom zumindest teilweise unter Erhalt eines Dimethylether, Kohlenstoffdioxid, Methanol und Wasser enthaltenden Rohproduktstroms einer direkten katalytischen Umsetzung zu Dimethylether unterworfen, wobei der Produktstrom unter Verwendung einer Produktaufbereitung gebildet wird, der zumindest ein Teil des Rohproduktstroms zugeführt wird. Das erfindungsgemäße Verfahren ist dadurch gekennzeichnet, dass der Eduktstrom der katalytischen Umsetzung mit einer Stöchiometriezahl von weniger als 2,0 zugeführt wird, dass der Produktstrom als zumindest Dimethylether und Kohlenstoffdioxid enthaltender Flüssigstrom gebildet wird, und dass der Produktstrom unter Verwendung zumindest eines Teils einer Sumpfflüssigkeit einer in der Produktaufbereitung verwendeten Trennkolonne bereitgestellt wird.

Zur Synthese von Dimethylether ist Kohlenstoffmonoxid als eines der Edukte erforderlich. Optional kann der erfindungsgemäß eingesetzte Eduktstrom jedoch zunächst auch überwiegend oder ausschließlich Kohlenstoffdioxid und nur geringe Mengen oder kein Kohlenstoffmonoxid enthalten. In diesem Fall kann das Kohlenstoffdioxid in dem Eduktstrom auch in situ in Kohlenstoffmonoxid umgesetzt werden, indem dieses beispielsweise mit dem enthaltenen Wasserstoff in eine Reaktionszone geführt werden, die einen Katalysator enthält, der die bereits erläuterte reverse Wassergasshift-Reaktion katalysiert. Dadurch wird in dieser Reaktionszone Kohlenstoffmonoxid gebildet. Die Reaktionszone, in der diese reverse Wassergasshift-Reaktion durchgeführt wird, kann vorteilhafterweise innerhalb einer Reaktionszone liegen, in der auch die Reaktion von Kohlenstoffmonoxid und Wasserstoff zu Methanol katalysiert wird. Insbesondere kann es sich um die gleiche Reaktionszone handeln, bei geeigneter Wahl des Katalysators kann ggf. auch derselbe Katalysator beide Reaktionen katalysieren.

Ein dem Verfahren zugeführter Eduktstrom kann daher beispielsweise Wasserstoff und Kohlenstoffdioxid, jedoch kein Kohlenstoffmonoxid enthalten, oder Wasserstoff und Kohlenstoffmonoxid, jedoch kein Kohlenstoffdioxid enthalten, oder Wasserstoff und sowohl Kohlenstoffmonoxid als auch Kohlenstoffdioxid enthalten.

Das erfindungsgemäße Verfahren ermöglicht einerseits die Bereitstellung von Mischungen aus Dimethylether und Kohlenstoffdioxid sowie ggf. weiteren Komponenten, wie sie für bestimmte Anwendungen erwünscht ist, andererseits verringert dies den Aufwand zur Trennung eines in der katalytischen Umsetzung erhaltenen Rohproduktstroms, da Kohlenstoffdioxid bezüglich relevanter thermischer Eigenschaften näher an Dimethylether liegt als dies für andere abzutrennende Stoffe, wie beispielsweise Methanol und Wasser, der Fall ist.

Weil die Stöchiometriezahl in dem Eduktstrom einen Wert von 2,0 erfindungsgemäß unterschreitet, kann Kohlenstoffdioxid in der erwünschten Konzentration direkt produziert werden, während eine Menge an Wasser als unerwünschtes Nebenprodukt reduziert wird.

Insbesondere werden der Trennkolonne Dimethylether und Kohlenstoffdioxid in Einsatzströmen zugeführt, die in der Produktaufbereitung unter Verwendung des Rohproduktstroms gebildet werden. Dadurch kann die Trennlast der Trennkolonne minimiert werden, da bereits angereicherte Ströme als die Einsatzströme verwendet werden. In einigen Ausgestaltungen wird dabei der Produktstrom als ein weniger als 0,1 mol-% (ggf. auch weniger als 0,05 mol-%) Methanol und Wasser enthaltender Flüssigstrom gebildet, was insbesondere für gegenüber Methanol und/oder Wasser intolerante Anwendungen vorteilhaft ist.

Alternativ dazu kann der Produktstrom als ein bis zu 5% Methanol und/oder bis zu 10% Wasser enthaltender Flüssigstrom gebildet werden, wenn der Produktstrom in einer gegenüber Methanol und Wasser toleranteren Anwendung verwendet werden soll. Dadurch kann die Ausbeute erhöht und der Trennaufwand gesenkt werden. In einigen Ausgestaltungen kann dabei die Produktaufbereitung eine Dehydratisierung von Methanol umfassen, was die Ausbeute an Dimethylether weiter erhöht und zugleich die Anforderungen an die Methanoltoleranz bei der Verwendung des Produktstroms senkt.

Insbesondere können dabei Dimethylether und Wasser, die in der Dehydratisierung gebildet werden, zumindest teilweise der Trennkolonne zugeführt werden, so dass diese ohne zusätzliche Investitionskosten in den Produktionskreislauf integriert werden können.

Die Produktaufbereitung wird dabei bevorzugt so betrieben, dass der Produktstrom zumindest 25 %, 50 %, 75 %, 90 %, 95 % oder 99 % des in dem Rohproduktstrom enthaltenen Dimethylethers und/oder des in dem Rohproduktstrom enthaltenen Kohlenstoffdioxids enthält. Dadurch können Ausbeute und Reinheit der abgegebenen Produkte gezielt eingestellt werden.

In der Produktaufbereitung wird bevorzugt zumindest ein Reststrom gebildet, der zumindest eines aus der Gruppe aus Wasserstoff, Kohlenstoffmonoxid und Methanol enthält und/oder der zumindest teilweise zu der katalytischen Umsetzung und/oder zu der Dehydratisierung zurückgeführt und dieser unterworfen wird. Dies erhöht die Gesamteffizienz des Verfahrens und stellt die erforderliche Produktspezifikation sicher. Insbesondere Methanol ist in der Regel nur in sehr geringen Mengen (z.B. im einstelligen Prozentbereich) in dem Produktstrom erwünscht bzw. tolerabel.

Der Eduktstrom kann vorteilhafterweise zumindest teilweise unter Verwendung einer Elektrolyse von Wasser und/oder Kohlenstoffdioxid und/oder einer Trockenreformierung von kohlenstoffhaltigem Ausgangsmaterial bereitgestellt werden. Damit sind regenerative Quellen für Kohlenstoff erschließbar, was sich im Lichte der Anstrengungen zur Decarbonisierung der globalen Wirtschaft auch finanziell positiv auswirkt.

Insbesondere kann der Eduktstrom neben Wasserstoff und Kohlenstoffmonoxid zumindest 5 %, 10 %, 20 %, 25 % oder mehr als 30 % (bis zu 100% des Kohlenstoffoxidanteils) Kohlenstoffdioxid enthalten. Damit kann auch extern verfügbares Kohlenstoffdioxid in den Produktstrom überführt und somit einer weiteren Nutzung zugeführt werden. Wie bereits erwähnt, kann ein von außen in das Verfahren eingeführter Strom oder der Eduktstrom selbst (anfänglich) auch arm an oder sogar frei von Kohlenstoffmonoxid sein, wobei das Kohlenstoffmonoxid, das für die Methanolsynthese benötigt wird, in einem solchen Fall beispielsweise über eine reverse Wassergasshift-Reaktion aus Kohlenstoffdioxid und Wasserstoff hergestellt wird.

Eine erfindungsgemäße Anlage zur Herstellung von Dimethylether umfasst Mittel, die die Anlage zur Durchführung eines wie vorstehend beschriebenen Verfahrens ertüchtigen. Die erfindungsgemäße Anlage bzw. deren vorteilhafte Weiterbildungen und Ausgestaltungen profitieren dementsprechend von den Vorteilen des jeweils entsprechenden Verfahrens in analoger Weise und umgekehrt.

Im Folgenden werden weitere Merkmale und Vorteile der Erfindung anhand eines Ausführungsbeispiels unter Bezugnahme auf die beiliegende Zeichnung näher erläutert.

Kurze Beschreibung der Figuren
Figur 1 zeigt eine vorteilhafte Ausgestaltung eines erfindungsgemäßen Verfahrens in stark vereinfachter Darstellung als Blockdiagramm.
Figur 2 zeigt eine vorteilhafte Ausgestaltung einer erfindungsgemäßen Anlage in Form einer stark vereinfachten Skizze besonders relevanter Funktionselemente.

### Ausführungsform(en) der Erfindung

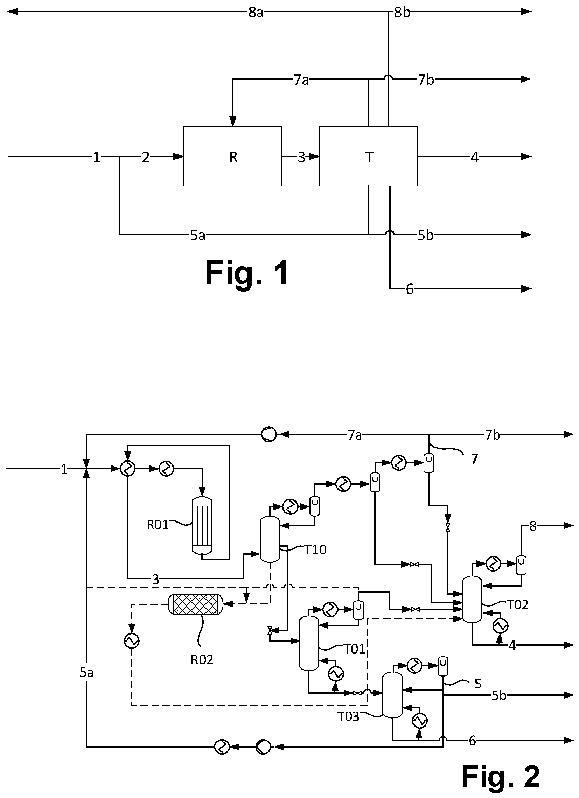

In Figur 1 ist eine vorteilhafte Ausgestaltung eines Verfahrens zur Herstellung von Dimethylether (DME) auf Basis von Synthesegas gezeigt. In Figur 2 ist eine Ausgestaltung einer entsprechenden Anlage schematisch dargestellt. Identische bzw. sich funktional entsprechende Stoffströme sind in den Figuren mit jeweils identischen Bezugszeichen referenziert.

Synthesegas 1 kann beispielsweise aus einer Dampfreformierung, Trockenreformierung, partiellen Oxidation, autothermen Reformierung, einem elektrochemischen Prozess wie die Ko-Elektrolyse, (Reverse) Wasser-Gas-Shift oder Kombinationsverfahren stammen. Auch ist die Vergasung von flüssigen und festen kohlenstoffhaltigen Einsätzen als Synthesegasquelle möglich. Alternativ kann statt herkömmlichem Synthesegas auch eine Mischung aus Wasserstoff und Kohlenstoffdioxid verwendet werden, aus der sich mittels RWGS Kohlenstoffmonoxid herstellen lässt.

Das Synthesegas 1 wird in einem Eduktstrom 2 zu einem Direktsynthesereaktor R, R01 geleitet. In der DME-Synthese setzt sich Wasserstoff (H₂) mit Kohlenstoffmonoxid (CO) gemäß folgender Reaktionsgleichungen über das Zwischenprodukt Methanol (MeOH) um:

2 H₂ + CO ↔ MeOH

2 MeOH ↔ DME + H₂O

CO + H₂O↔ CO₂ + H₂

Es handelt sich hierbei um Gleichgewichtsreaktionen, die je nach Druck (30-100 bar), Temperatur (200-300°C) und Zusammensetzung des Eduktstroms 2 eine andere Produktzusammensetzung ergeben. Bei einer Stöchiometriezahl (SN), also dem eingangs beschriebenen Verhältnis der Konzentrationen von Wasserstoff, Kohlenstoffmonoxid und Kohlenstoffdioxid, von 1 beobachtet man unter idealen Bedingungen (d.h. unter der Annahme, dass alle Kreisläufe geschlossen sind und alle Produkte ausgeleitet werden) die folgende Nettoreaktion:

3 H₂ + 3 CO → DME + CO₂

Bei einer SN von 2 beobachtet man idealisiert die folgende Nettoreaktion:

2 H₂ + CO → DME + H₂O

Weitere Ströme in der Anlage sind:
- ein DME Rohproduktstrom 3, der einer Trennung T unterworfen wird,
- ein DME Produktstrom 4, der je nach Trennsequenz eine entsprechende Reinheit aufweist (z.B. "fuel grade" mit >98,5 wt-% DME),
- ein MeOH-Strom 5 aus der Trennung T, der in einen MeOH-Recycle 5a und einen MeOH-Purge 5b aufgespalten wird,
- ein Abwasserstrom 6,
- ein Synthesegasstrom 7 aus der Trennung, der in einen Synthesegas-Recycle 7a und einen Synthesegas-Purge 7b aufgespalten wird, und
- ein CO₂-reicher Strom 8 aus der Trennung, der in einen CO₂-Recycle 8a und einen CO₂-Purge 8b aufgespalten wird

Die Trennung T ist in Figur 2 in größerer Detailtiefe anhand eines Beispiels gezeigt. Stromab des DME Synthesereaktors R01 wird der Rohproduktstrom 3 abgekühlt, z.B. durch Wärmeintegration mit dem Reaktionseinsatzgas bzw. Eduktstrom 2, und in eine erste Trennkolonne T10 geleitet. Das Sumpfprodukt der ersten Trennkolonne T10 wird in einer zweiten Trennkolonne T01 an DME und weiteren gelösten Gasen abgereichert, so dass ein (überwiegend) MeOH und H₂Oenthaltener Strom als Sumpfprodukt der zweiten Trennkolonne T01 entsteht. Das MeOH wird in einer dritten Trennkolonne T03 abgetrennt und zum DME Reaktor R01 rezykliert.

Wasser 6 wird aus dem System entfernt und kann beispielsweise zur Synthesegasgewinnung genutzt werden, insbesondere in Fällen, in denen dazu eine Elektrolyse verwendet wird.

Der Kopfstrom aus der ersten Trennkolonne T10 wird stufenweise abgekühlt, wodurch DME-reiche Kondensate entstehen. Die Kondensate werden in eine vierte Trennkolonne (T02, DME/CO2 Kolonne) eingeleitet. In der vierten Trennkolonne T02 wird als Sumpfprodukt DME, z.B. mit einer Kraftstoffspezifikation, erhalten. Am Kopf der vierten Trennkolonne T02 werden leichtere gelöste Gase wie H₂ und CO gesammelt. Dieser Strom kann auch gewisse Mengen an CO₂ enthalten und wird bevorzugt teilweise kondensiert und das nicht-kondensierte Gas kann zum DME-Reaktor R01 rezykliert (7a) oder gepurged (7b), also aus der Anlage ausgeschleust, werden (dies ist der Fall, wenn CO₂ aus dem System entfernt werden soll). In herkömmlichen Verfahren, die nicht Teil dieser Erfindung sind, wird die vierte Trennkolonne T02 typischerweise so betrieben, dass möglichst hohe Anteile des in dem Rohproduktstrom 03 enthaltenen CO₂ abgetrennt werden und nicht in den Produktstrom 4 überführt werden.

Um demgegenüber ein Stoffgemisch aus CO₂ und DME als den Produktstrom 4 bereitzustellen wird in Ausgestaltungen des erfindungsgemäßen Verfahrens die vierte Trennkolonne T02 so betrieben, dass ein erheblicher Teil des in dem Rohproduktstrom 3 enthaltenen CO₂ in den Produktstrom 4 überführt wird. Der Reaktor R01 wird dabei entgegen herkömmlicher Vorgehensweisen mit einer Stöchiometriezahl SN von kleiner als 2 betrieben, um Kohlenstoffdioxid Verfahrensintern zu erzeugen. In anderen Ausgestaltungen kann aber auch vorgesehen sein, dass der dem Reaktor R01 zugeführte Eduktstrom bereits nennenswerte Anteile an CO₂ enthält, die den Reaktor R01 unverändert passieren, ohne die darin ablaufenden Reaktionen zu stören. Insbesondere kann durch einen so erhöhten Anteil an nicht an den Reaktionen teilnehmenden Substanzen in dem Eduktstrom die thermische Leistung des Reaktors R01 gesteuert werden, so dass eine Kühlung des Reaktors R01 leistungsschwächer ausgelegt werden kann, als dies in herkömmlichen Verfahren der Fall ist. Dadurch kann eine Effizienzsteigerung der gesamten Anlage erreicht werden.

Die Anpassung des Betriebs der vierten Trennkolonne T02 im Vergleich zu herkömmlichen Verfahren kann im Wesentlichen durch eine Anpassung des Druck-und/oder Temperaturregimes und/oder des Rücklaufverhältnisses erfolgen, so dass eine Löslichkeit von CO₂ in DME und/oder ein Anteil von flüssigem CO₂ am gesamten CO₂-Anteil des in der vierten Trennkolonne T02 bearbeiteten Stoffgemisches erhöht wird. Dies kann insbesondere durch Temperaturverringerung und/oder Druckerhöhung und/oder Erhöhung des Rücklaufverhältnisses (zur Erhöhung des Anteils an gelöstem CO₂) im Vergleich zu herkömmlichen Vorgehensweisen bewirkt werden.

Je nach Anforderungen an die Zusammensetzung des Produktstroms 4 kann ggf. auf die dritte T03 und/oder die zweite T01 Trennkolonne verzichtet werden. Vorteilhafterweise wird dann jedoch ein MeOH-Dehydratisierungsreaktor R02 zwischen der ersten Trennkolonne T10 und der vierten Trennkolonne T02 im Pfad des Sumpfstroms der ersten Trennkolonne T10 angeordnet, um das in dem Sumpfstrom der ersten Trennkolonne T10 enthaltene Methanol zumindest teilweise in Dimethylether und Wasser umzusetzen und so die Gesamtausbeute des Verfahrens zu erhöhen. In derartigen Ausgestaltungen kann der Produktstrom neben CO₂ und Dimethylether auch Methanol und Wasser enthalten. Dies ist für bestimmte Anwendungen jedoch unschädlich bzw. akzeptabel und ermöglicht somit erhebliche Einsparungen sowohl im Bereich der Investitionskosten als auch bei den Betriebskosten der entsprechenden Anlage. Durch diesen MeOH-Dehydratisierungsreaktor R02 wird der MeOH Anteil im Produktstrom 4 signifikant reduziert. Eine vorangehende Abtrennung des Wassers sorgt für einen hohen Umsatz im MeOH-Dehydratisierungsreaktor. Sofern ein erhöhter Wasser- und MeOH-Anteil im Produkt akzeptabel ist, kann eine weitere Kolonne (T01) eingespart werden.

Durch entsprechende Installation von Umgehungsleitungen und Ventilen können auch bestehende Anlagen so umgerüstet werden, dass sie je nach gewünschter Zusammensetzung des Produktstroms wahlweise mit den zweiten und/oder dritten Trennkolonnen T01, T03 oder unter Umgehung einer oder beider dieser Trennkolonnen T01, T03 betrieben werden können. Entfallen eine oder beider der zweiten und dritten Trennkolonnen T01, T03, so entfallen gegebenenfalls auch die entsprechenden dort gebildeten Ströme 5 (5a, 5b) und 6 bzw. werden gemeinsam - ggf. nach entsprechender Bearbeitung in einem optionalen MeOH-Dehydratisierungsreaktor R02 - in die vierte Trennkolonne T02 transferiert.

Im Gegenzug zum DME-Direktsynthese-Reaktor R01, der gekühlt werden muss (Ausführung z.B. als Linde-Isothermreaktor, Rohrbündelreaktor), kann der MeOH-Dehydratisierungsreaktor R02 adiabat ausgeführt werden. Die MeOH-Dehydratisierungsreaktion (2 MeOH ↔ DME + H2O) ist eine gleichgewichtslimitierte Reaktion und findet in der Gasphase bei ~400°C an sauren, heterogenen Katalysatoren statt.

## Patentansprüche

1. Verfahren zur Herstellung eines Dimethylether enthaltenden Produktstroms (4),
wobei ein zumindest Wasserstoff und ein oder mehrere Kohlenstoffoxide aus der Gruppe von Kohlenstoffmonoxid und Kohlenstoffdioxid enthaltender Eduktstrom (2) zumindest teilweise unter Erhalt eines Dimethylether, Kohlenstoffdioxid, Methanol und Wasser enthaltenden Rohproduktstroms (3) einer direkten katalytischen Umsetzung (R, R01, R02) zu Dimethylether unterworfen wird, und
wobei der Produktstrom (4) unter Verwendung einer Produktaufbereitung (T, T10, T01, T02, T03) gebildet wird, der zumindest ein Teil des Rohproduktstroms (3) zugeführt wird,
**dadurch gekennzeichnet,**
**dass** der Produktstrom (4) als zumindest Dimethylether und Kohlenstoffdioxid enthaltender Flüssigstrom gebildet wird, und
**dass** der Produktstrom (4) unter Verwendung zumindest eines Teils einer Sumpfflüssigkeit einer in der Produktaufbereitung (T, T10, T01, T02, T03) verwendeten Trennkolonne (T02) bereitgestellt wird.

2. Verfahren nach Anspruch 1, wobei der Eduktstrom (2) der katalytischen Umsetzung (R, R01, R02) mit einer Stöchiometriezahl von weniger als 2,0 zugeführt wird.

3. Verfahren nach Anspruch 1 oder 2, bei dem der Eduktstrom Kohlenstoffdioxid enthält, das in einer Wassergas-Shiftreaktion mit Wasserstoff zumindest zum Teil zu Kohlenstoffmonoxid umgesetzt wird.

4. Verfahren nach einem der vorstehenden Ansprüche, wobei der Trennkolonne (T02) Dimethylether und Kohlenstoffdioxid in Einsatzströmen zugeführt werden, die in der Produktaufbereitung (T, T10, T01, T02, T03) unter Verwendung des Rohproduktstroms (3) gebildet werden.

5. Verfahren nach Anspruch 4, wobei der Produktstrom (4) als ein weniger als 0,1 mol-% Methanol und Wasser enthaltender Flüssigstrom gebildet wird.

6. Verfahren nach Anspruch 4, wobei der Produktstrom (4) als ein bis zu 5% Methanol und/oder bis zu 10% Wasser enthaltender Flüssigstrom gebildet wird.

7. Verfahren nach Anspruch 6, wobei die Produktaufbereitung (T, T10, T01, T02, T03) eine Dehydratisierung von Methanol umfasst.

8. Verfahren nach Anspruch 7, wobei Dimethylether und Wasser, die in der Dehydratisierung (R02) gebildet werden, zumindest teilweise der Trennkolonne (T02) zugeführt werden.

9. Verfahren nach einem der vorstehenden Ansprüche, wobei in der Produktaufbereitung (T, T10, T01, T02, T03) zumindest ein Reststrom (5, 6, 7, 8) gebildet wird, der zumindest eines aus der Gruppe aus Wasserstoff (7), Kohlenstoffmonoxid (7) und Methanol (5) enthält und/oder der zumindest teilweise zu der katalytischen Umsetzung (R, R01) und/oder zu der Dehydratisierung (R02) zurückgeführt (5a, 7a) und dieser bzw. diesen unterworfen wird.

10. Verfahren nach einem der vorstehenden Ansprüche, wobei der Eduktstrom (2) zumindest teilweise unter Verwendung einer Elektrolyse von Wasser und/oder Kohlenstoffdioxid und/oder einer Trockenreformierung und/oder partiellen Oxidation und/oder autothermen Reformierung von kohlenstoffhaltigem Ausgangsmaterial bereitgestellt wird.

11. Verfahren nach einem der vorstehenden Ansprüche, wobei der Eduktstrom (2) neben Wasserstoff und Kohlenstoffmonoxid zumindest 5 %, 10 %, 20 %, 25 % oder mehr als 30 % Kohlenstoffdioxid enthält.

12. Anlage zur Herstellung von Dimethylether, die Mittel (T01, T02, T03, T10, R02) umfasst, die die Anlage zur Durchführung eines Verfahrens nach einem der Ansprüche 1 bis 11 ertüchtigen.
